# EUROPÄISCHE PATENTSCHRIFT

(11) **EP 2 416 720 B1**
(45) Veröffentlichungstag und Bekanntmachung des Hinweises auf die Patenterteilung: **08.02.2017**
(21) Anmeldenummer: 10720246.7
(22) Anmeldetag: 30.03.2010
(51) Int. Cl.: A61B 17/80, A61B 17/68

(54) **VORRICHTUNG ZUR WINKELSTABILEN FIXATION UND KOMPRESSION EINER BRUCHSTELLE BZW. OSTEOTOMIE AN EINEM KNOCHEN**
APPARATUS FOR THE CONSTANT-ANGLE FIXATION AND COMPRESSION OF A FRACTURE OR OSTEOTOMY OF A BONE
DISPOSITIF DE FIXATION À STABILITÉ ANGULAIRE ET DE COMPRESSION D'UN FOYER DE FRACTURE OU D'UNE OSTÉOTOMIE SUR UN OS

(30) Priorität: 07.04.2009 DE 102009016394
(43) Veröffentlichungstag der Anmeldung: 15.02.2012
(73) Patentinhaber: Aristotech Industries GmbH, 14943 Luckenwalde (DE)
(72) Erfinder: HILSE, Martin, 12105 Berlin (DE); KRANZ, Curt, 14163 Berlin (DE); ANAPLIOTIS, Emmanuel, 14195 Berlin (DE)
(74) Vertreter: Hannig, Wolf-Dieter
(86) Internationale Anmeldenummer: PCT/DE2010/000365
(87) Internationale Veröffentlichungsnummer: WO 2010/115403

(56) Entgegenhaltungen:
- EP-A1- 2 016 918
- EP-A2- 1 468 655
- WO-A1-02/096309
- US-A1- 2008 132 955

## Beschreibung

Die Erfindung betrifft eine Vorrichtung zur winkelstabilen Fixation und Kompression einer Bruchstelle bzw. Osteotomie an einem Knochen, mit einer Knochenplatte, die aus einer Oberseite, einer Unterseite und mehreren die Ober- und Unterseite verbindenden, in Längsachse der Platte angeordneten Löchern gebildet ist, und Knochenschrauben mit Gewindekopf, die in den Löchern aufgenommen und im Knochen verschraubt sind, wobei mindestens ein Loch eine Kombination aus zwei kreisrunden Löchern mit unterschiedlich großen Durchmessern ist und die Symmetriezentren der beiden Löcher auf der Längsachse der Knochenplatte angeordnet sind, wobei die kreisrunden Löcher so gegeneinander in Richtung Längsachse verschoben angeordnet sind, dass ein Kreisabschnitt des kleinen Lochs am Umfang des größeren Loches eine durch Kanten abgesetzte, einseitige sichelartige Erweiterung bildet.

Aus der EP 1 255 498 B1 ist eine Knochenplatte bekannt, welche entweder für eine Kompression oder eine Fixation bei der Versorgung von Frakturen des Knochens eingesetzt werden kann. Dies erreicht dieser bekannte Stand der Technik dadurch, dass die Knochenplatte eine Oberseite, einer für den Knochenkontakt bestimmten Unterseite sowie mehreren die Ober- und Unterseite verbindenden, entlang der Plattenlängsachse angeordneten Löchern für die Aufnahme von Knochenschrauben hat, wobei mindestens eines der Löcher aus einer Kombination eines kreisförmigen Loches mit einem Langloch besteht. Der Abstand zwischen den Symmetriezentren des kreisförmigen Loches und des Langloches ist dabei kleiner als die Summe aus dem Radius des kreisförmigen Loches und der Hälfte der Langachse des Langloches. Unter einem Langloch definiert dieser bekannte Stand der Technik ein ovales, ellipsenförmiges, ein rechteckiges Loch oder eine Kombination solcher Formen als Langloch, jedoch werden kreisrunde Löcher davon ausgeschlossen.
Dieser bekannte Stand der Technik kann entweder nur eine Kompression oder eine Fixation erzeugen. Die Kompression wird beim Festziehen der Knochenschraube im Langloch durch Längsverschiebung der Frakturelemente erreicht. Eine gleichzeitige winkelstabile Fixation bewirkt dies aber nicht. Diese lässt sich nur durch eine gesonderte Knochenschraube in einem anderen Langloch erzielen, so dass eine Vielzahl von Knochenschrauben zum Einsatz gelangen muss. Die Versorgung der Fraktur ist aufwändig und verkompliziert den Eingriff beim Patienten.

Weiterhin ist aus der DE 31 13 639 A1 eine osteosynthetische Kompressionsplatte mit mehreren in Längsrichtung der Platte angeordneten schlitzartigen Löcher mit Auflageflächen für Senk-Knochenschrauben mit Kugelkopf bekannt. Die Kompression der Fraktur wird hier durch eine Verschiebebewegung der beiden Knochenteile erreicht, in dem der Kugelkopf der Senk-Knochenschraube entlang einer kegeligen oder kugeligen Ansenkung innerhalb der schlitzartigen Löcher in Längsrichtung der Platte gleitet. Die Fixation in der so komprimierten Lage geschieht durch eine weitere Knochenschraube. Insofern unterscheidet sich dieser Stand der Technik nicht von dem zuvor erwähnten.

Die WO 02/096309 A1 beschreibt eine Knochenplatte zur Fixation von proximalen Humerusfrakturen mit einem stilförmigen Teil, dessen Länge größer als seine Breite ist, und einem löffelartigen Teil, dessen Breite größer als die Breite des stilförmigen Teils ist, wobei der stilförmige und der löffelförmige Teil eine gemeinsame longitudinale Zentralachse aufweisen. Die Löcher im stilförmigen Teil bestehen aus einer Kombination eines Loches mit einem größeren und einem Loch mit einem kleineren Durchmesser, wobei mindestens eines der Schraubenlöcher ein mindestens partielles Innengewinde aufweisen.
Auch mit diesem bekannten Stand der Technik ist eine gleichzeitige Kompression und Fixation innerhalb eines Schraubenloches nicht möglich.
Des Weiteren ist aus der EP 1 468 655 A2 eine Knochenplatte zur Verwendung bei der Osteotomie bekannt, wobei die Knochenplatte mindestens eine Längsachse, eine mit den Knochen in Berührung stehende Unterseite und eine Oberseite mit Öffnungen, die durch die Platte von der Oberseite zur Unterseite miteinander in Verbindung stehen, aufweist, wobei die Öffnungen zur Aufnahme von Knochenstiften oder Knochenschrauben dienen und die Öffnungen im Wesentlichen entlang der Längsachse angeordnet sind, worin zwei dieser Öffnungen als ein Satz von zwei überlappenden Löchern ausgeführt sind und worin mindestens zwei Löcher entlang einer zweiten Achse ausgerichtet sind, die sich in einem Winkel zur Längsachse erstreckt. Die beiden überlappenden Öffnungen des einen Satzes von überlappenden Löchern sind zylinderförmig und mit Gewinde und einer Anfasung versehen. Dieser bekannte Stand der Technik ermöglicht es nur, die Knochenschraube an der angefasten Oberfläche zu klemmen bzw. verkeilen. Eine Verschiebung der Platte beim Einschrauben ist nicht möglich, weil die Einschraubkraft an den angefasten Oberflächen von Schraubenkopf und Loch in eine von der Bruchstelle weg gerichtete größere Kraftkomponente und eine in Richtung Bruchstelle gerichtete kleinere Kraftkomponente zerfällt. Die einseitige Kompression der Bruchstelle wird durch den Chirurgen mit der Wahl des Einschraubpunktes der Knochenschraube im Loch bewirkt. Die Herstellung der Knochenplatte ist durch die unterschiedlichen Anfasungen der sich Löcher entsprechend aufwändig.
Weiterhin ist aus der EP 2 016 018 A1 eine geblockte Platte mit Löchern gleichen Durchmessers mit einer abwärts verlaufenden sphärischen Anfasung, die mit dem runden Kopf einer Knochenschraube zusammenwirkt und das Klemmen bewirkt, so dass auch bei diesem Stand der Technik die Fixation und Kompression auseinanderfallen.
Auch beim Stand der Technik nach US 2008/0132955 A1 wird die Kompression durch ein Klemmen des Kopfes der Knochenschraube an einer Anfasung erzielt und die Fixation mittels der Verschraubung.

### Aufgabenstellung

Bei diesem Stand der Technik liegt der Erfindung die Aufgabe zugrunde, eine Vorrichtung zur winkelstabilen Fixation und Kompression einer Bruchstelle bzw. Osteotomie an einem Knochen anzugeben, die es ermöglicht, die winkelstabile Fixation und die Kompression der Bruchstelle zu vereinfachen und beide Funktionen gleichzeitig mit einer einzigen Knochenschraube zu erreichen.

Diese Aufgabe wird durch eine Vorrichtung der eingangs genannten Gattung mit den Merkmalen des Anspruches 1 gelöst.

Vorteilhafte Ausgestaltungen des erfindungsgemäßen Vorrichtung sind den Unteransprüchen entnehmbar

Die erfindungsgemäße Lösung zeichnet sich dadurch aus, dass die Befestigungs- und Kompressionsfunktion innerhalb eines Schraubenloches von einer einzigen Knochenschraube wahrgenommen werden kann. Dies ist mit dem außerordentlichen Vorteil für den Patienten verbunden, dass die Anzahl der Knochenschrauben zum Versorgen der Fraktur erheblich reduziert werden kann, der Knochen weniger perforiert wird, die Abmessungen der Knochenplatten verringert und der operative Eingriff vereinfacht werden kann.

Die Zusammenführung der Befestigungs- und Kompressionsfunktion in einer Schraube gelingt dadurch, dass die Innenwandung des großen Loches mit einem von der Oberseite zur Unterseite verlaufenden Innengewinde versehen ist, das sich bis an die Kanten erstreckt, und dass die sichelartige Erweiterung ohne Gewinde ausgebildet ist und das Symmetriezentrum des kleinen Loches nahe am Symmetriezentrum, des großen Loches angeordnet ist, wobei der Abstand der beiden Symmetriezentren die Bedingung 1/18 D2 < A < 1/3 D2 erfüllt, wobei die Knochenschraube den Knochen im Symmetriezentrum des kleinen Loches durch Einschrauben fixiert und zugleich der Gewindekopf ein konisches Außengewinde aufweist, das beim Einschrauben das Innengewinde des großen Loches erfasst und eine relative Verschiebung der Platte zum Knochen erzeugt.

Von besonderem Vorteil ist weiterhin, dass das größere Loch mit seinem Innengewinde zur Bruchstelle des Knochens hin und die sichelförmige Erweiterung jeweils von der Bruchstelle weg angeordnet sind, so dass es möglich wird, die Fixation und Kompression mit sehr wenig Bauelementen auf kleinstem Raum zu bewerkstelligen.

Die Achsen des Loches mit dem großen Durchmesser und der sichelförmigen Erweiterung sind im Wesentlichen senkrecht zur Oberseite der Knochenplatte angeordnet und verlaufen zueinander parallel. Dies vereinfacht nicht nur die Herstellung der Knochenplatten, sondern auch die Befestigung auf dem entsprechenden Knochenteil.

In einer bevorzugten Ausführungsform beträgt das Verhältnis der Durchmesser zwischen dem Loch mit dem großen Durchmesser und der sichelförmigen Erweiterung mit dem kleinen Durchmesser 0,5 < D2/D1 < 0,8. Dies stellt sicher, dass sich die Symmetriezentren des Loches mit dem großen Durchmesser und der sichelförmigen Erweiterung mit dem kleinen Durchmesser nahe zueinander angeordnet sind, so dass die Steifigkeit und Festigkeit der Knochenplatte infolge des geringeren Platzbedarfs für die Löcher verbessert und auch die Dicke der Knochenplatte verringert werden kann.

Bei einer weiteren bevorzugten Ausführungsform der Erfindung ist das Innengewinde des großen Loches ein kreiszylindrisches Feingewinde, dessen Steigung im Bereich zwischen 0,5 mm und 1,2 mm liegt. Das konische Außengewinde des Gewindekopfes weist dazu einen Konuswinkel von 10° und 20°, vorzugsweise 16° auf. Dies gewährleistet eine feinfühlige Einstellung der Kompression auf die im jeweiligen Anwendungsfall erforderlichen Werte.

In einer weiteren bevorzugten Ausgestaltung wird die erfindungsgemäße Vorrichtung durch eine Bohrlehre zum Setzen einer Bohrung im Symmetriezentrum des Loches mit dem kleinen Durchmesser im Knochen ergänzt, so dass die gewünschte orthogonale Lage zur Längsachse der Knochenplatte einfach und problemlos eingehalten werden kann.

Bevorzugte Materialien für die Knochenplatte und Knochenschraube sind vorzugsweise biokompatible Werkstoffe wie Titan, Titanlegierungen, Stahl, Kobalt-Chrom-Legierungen, Kunststoff oder Composite. Es gehört auch zur Erfindung, dass die Knochenplatte und die Knochenschraube jeweils aus verschiedenen Materialien mit unterschiedlichen mechanischen Eigenschaften bestehen können.

Weitere Vorteile und Einzelheiten ergeben sich aus der nachfolgenden Beschreibung unter Bezugnahme auf die beigefügten Zeichnungen.

### Ausführungsbeispiel

Die Erfindung soll nachstehend an einem Ausführungsbeispiel näher erläutert werden.

### Es zeigt

Fig. 1 eine perspektivische Ansicht der Knochenplatte der erfindungsgemäßen Vorrichtung mit Darstellung der Lage der Löcher in der Knochenplatte,
Fig. 2 eine vergrößerte Darstellung der aus einem Loch mit größerem Durchmesser und einem Loch mit kleinerem Durchmesser gebildeten Fixations- und Kompressionsloch in vergrößerter Ansicht,
Fig. 3 einen Schnitt der Knochenplatte mit im Knochen eingesetzten Knochenschrauben im Zustand vor dem Eingriff des Außengewindes des Gewindekopfes der Knochenschraube in das Innengewinde des Loches mit dem größeren Durchmesser,
Fig. 4 eine Draufsicht nach Fig. 3,
Fig. 5 einen Schnitt der Knochenplatte mit im Knochen eingesetzten Knochenschrauben im Zustand nach dem vollständigen Einschrauben des Außengewindes des Gewindekopfes der Knochenschraube in das Innengewinde des Loches mit dem großen Durchmesser und
Fig. 6 eine Draufsicht nach Fig. 5.

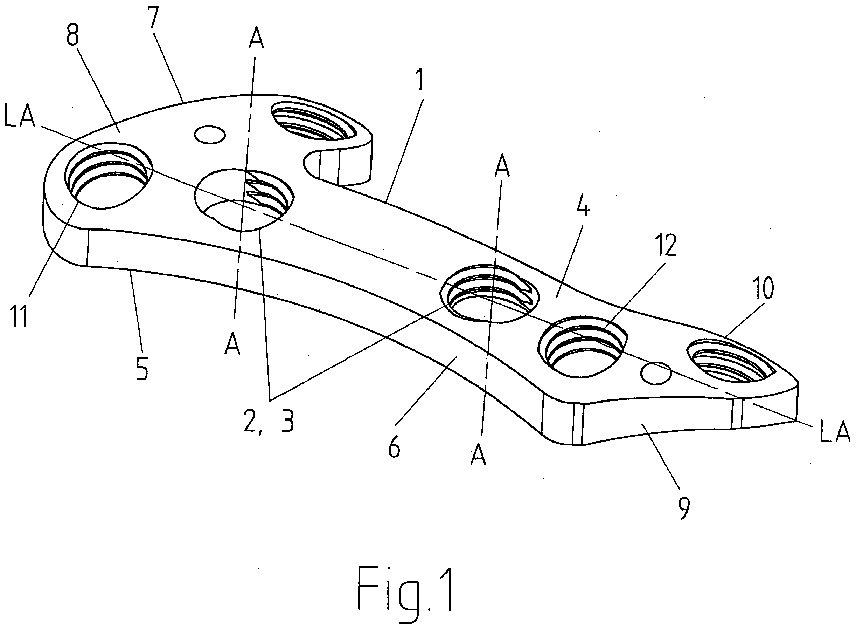

Die Fig. 1 zeigt die Knochenplatte 1 der erfindungsgemäßen Vorrichtung zum winkelstabilen Fixieren und zur Kompression einer Bruchstelle an einem Knochen mittels zweier gegenüberliegender Fixations- und Kompressionslöcher 2 und 3, die die Oberseite 4 und die Unterseite 5 der Knochenplatte 1 verbinden. Die Knochenplatte 1 besitzt eine Krümmung, die der Form und Gestalt der zu versorgenden Knochenteile angepasst ist. Die Fixations- und Kompressionslöcher 2 und 3 liegen auf einer gemeinsamen Längsachse LA des Stilteiles 6 der Knochenplatte 1. Ihre Achsen A sind im Wesentlichen senkrecht zur Längsachse A angeordnet und verlaufen zueinander parallel.

Der Stilteil 6 ist an seinem Ende 7 mit einer T-förmigen Verbreiterung 8 und an seinem anderen Ende 9 mit einer L-förmigen Verlängerung 10 versehen. Die Verbreiterung 8 und Verlängerung 10 weist jeweils zwei kreisförmige Löcher 11 mit zylindrischen Innengewinde 12 auf, die zur lagergerechten Fixierung der Knochenplatte 1 an den zu versorgenden Knochenteilen dienen. Es versteht sich, dass natürlich auch anderen Formen der Knochenplatte von der Erfindung erfasst sind.
Zur erfindungsgemäßen Vorrichtung gehören Knochenschrauben 13 und eine Bohrlehre, auf die im Abschnitt [0027] näher eingegangen wird.

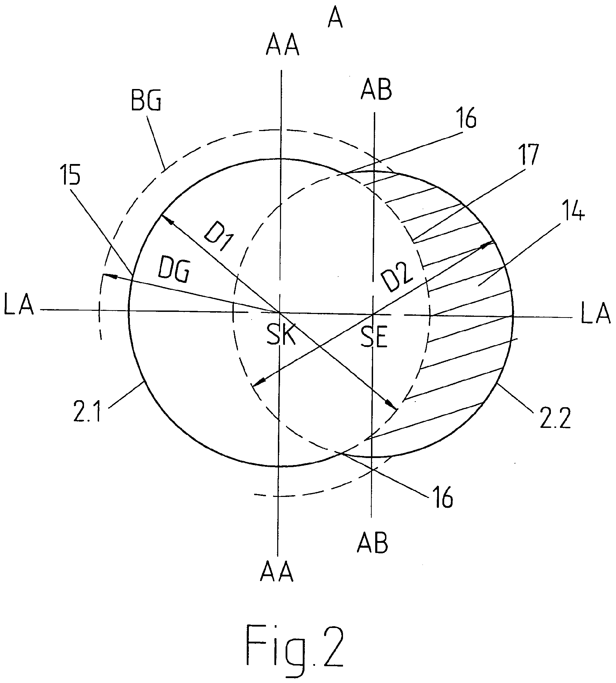

Die Fig. 2 zeigt das Fixations- und Kompressionsloch 2 in vergrößerter Ansicht mit Darstellung der geometrischen Verhältnisse bzw. Bedingungen.
Das Fixations- und Kompressionsloch 2 wird gebildet aus einem kreisförmigen Loch 2.1 mit dem Durchmesser D1 und einem kreisförmigen Loch 2.2 mit dem Durchmesser D2, die gegeneinander so verschoben sind, dass das kreisförmige Loch 2.1 eine sichelförmige Erweiterung 14 in Richtung weg vom Symmetriezentrum SK des Loches 2.1 aufweist. Der Durchmesser D2 ist gegenüber dem Durchmesser D1 kleiner. Beide Durchmesser D1 und D2 verhalten sich wie 0,5 < D2/D1 < 0,8.

Das Symmetriezentrun SK der Loches 2.1 und das Symmetriezentrum SE der Erweiterung 14 liegen dicht beieinander auf der Längsachse LA der Knochenplatte 1 und haben voneinander einen Abstand A, der die folgende geometrische Bedingung 1/18 D2 < A < 1/3 D2 erfüllt.

Das kreisförmige Loch 2.1 ist auf seiner Innenwand mit einem zylindrischen Innengewinde, insbesondere Feingewinde 15 versehen. Das Feingewinde 15 verläuft bis an die Kanten 16, an denen die Erweiterung 14 in das kreisförmige Loch 2.1 übergeht, so dass das Feingewinde 15 einen Kreisbogen BG beschreibt und gegenüber der Erweiterung 14 einen seitlich senkrecht verlaufenden offenen Teil 17 bildet.

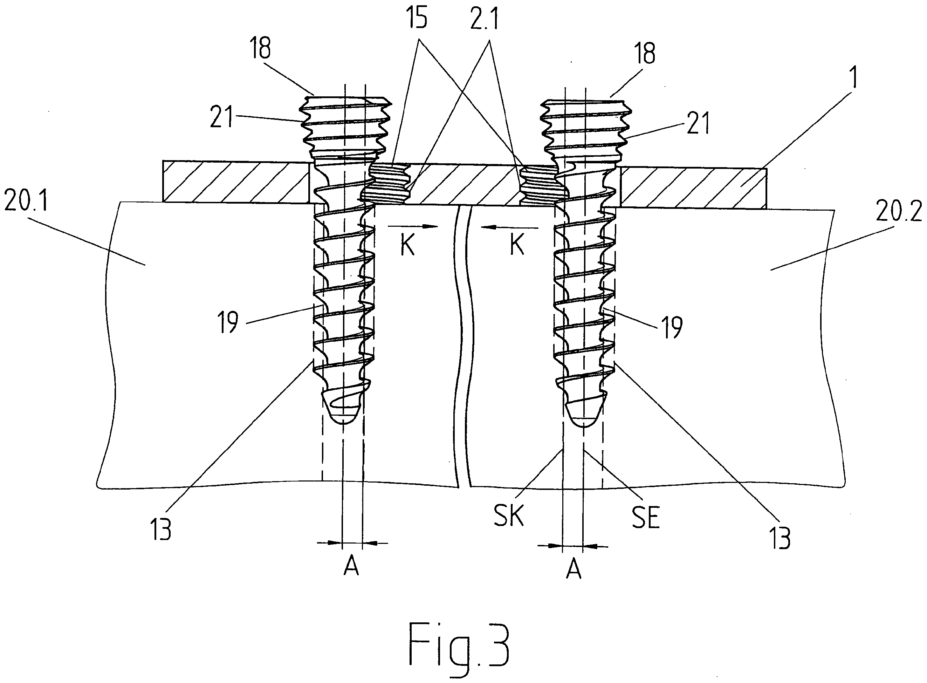

Die Fig. 3 zeigt einen Schnitt der Knochenplatte 1 im Zustand vor dem Einschrauben des Gewindekopfes 18 der Knochenschraube 13 in das Innengewinde 15 im kreisförmigen Loch 2.1. Die Knochenschraube 13 ist bereits teilweise in die Vorbohrungen 19 in den zur Fraktur gehörenden Knochenteilen 20.1 und 20.2 eingeschraubt. Die Vorbohrungen 19 werden mittels einer nicht dargestellten Bohrlehre im Symmetriezentrum SE der sichelförmigen Erweiterung 14 in die Knochenteilen 20.1 und 20.2 gesetzt. Der Gewindekopf 18 besitzt ein konisches Außengewinde 21, das auf das Innengewinde 15 im kreisförmigen Loch 2.1 abgestimmt ist. Der Konuswinkel α des Außengewindes liegt im Bereich von 10° bis 20°, vorzugsweise 16°. Zur Abstimmung des konischen Außengewindes auf das Innengewinde wird auf die WO 2007/025520 A1 verwiesen. Dadurch, dass die Symmetriezentren SK des kreisförmigen Loches 2.1 und SE der sichelförmigen Erweiterung 14 dicht aneinander angeordnet sind, kann beim weiteren Einschrauben das Außengewinde 21 der Knochenschraube 13 seitlich in den offenen Teil 17 des Innengewindes 15 eindringen und dieses erfassen, so dass im eingeschraubten Zustand der Gewindekopf 18 vollständig in dem Innengewinde 15 aufgenommen ist. Die Knochenschrauben 13 liegen in der Vorbohrung 19 fest.

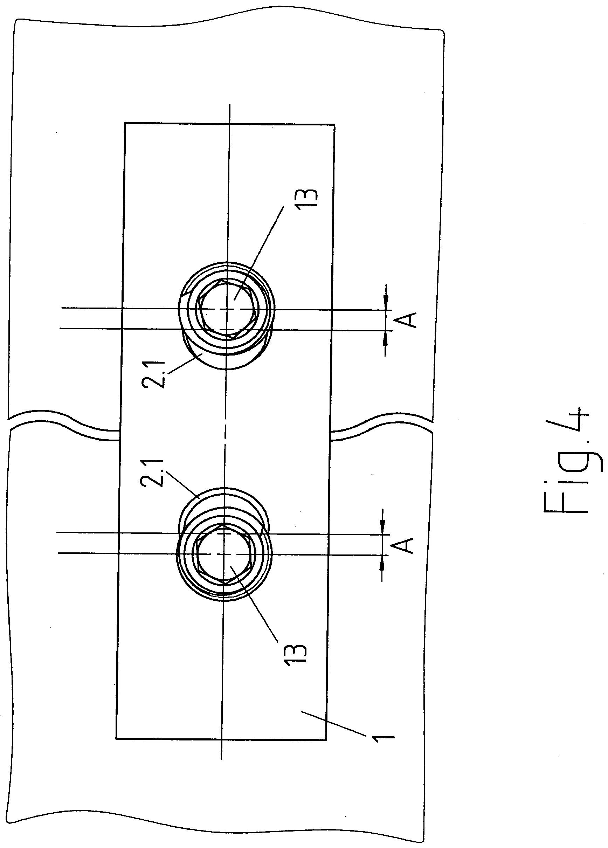

Die Fig. 4 zeigt diesen Einschraubzustand in der Draufsicht auf die Knochenplatte 1.

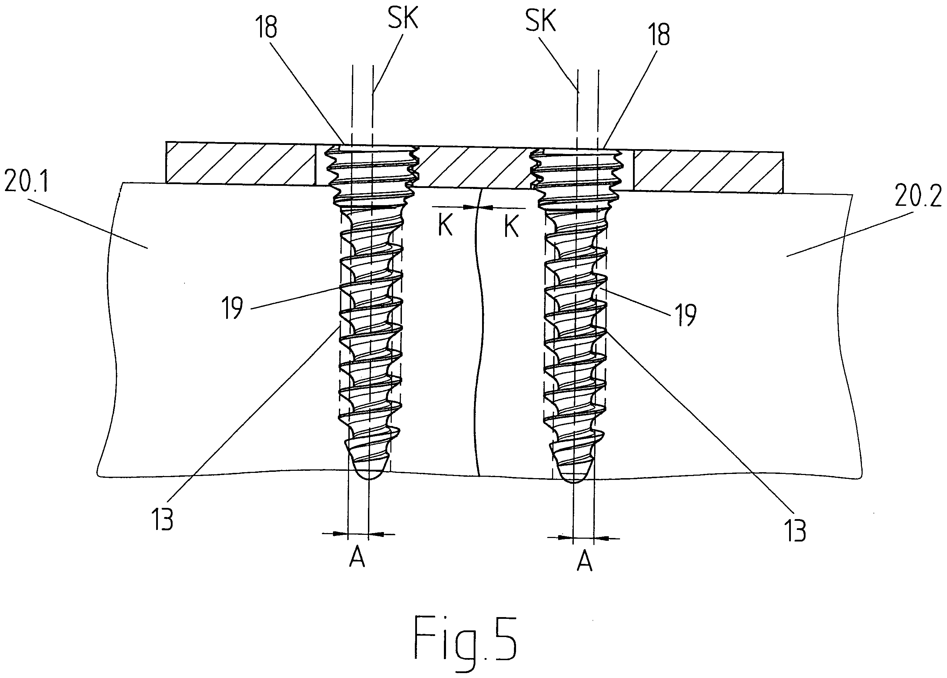

In der Fig. 5 sind die Knochenschrauben 13 im vollständig eingeschraubten Zustand gezeigt. Die konischen Gewindeköpfe 18 beider Knochenschrauben 13 erzeugen beim Einschrauben in das Innengewinde 15 des Loches 2.1 jeweils gegeneinander Zwangsbewegungen K in Richtung Bruchstelle, so dass diese nach Zusammenpressen der Knochen unter Kompression gehalten wird.
Die Knochenschraube 13 fixiert und übt auf die Knochenteile 20.1 und 20.2 über das konische Außenwindes des Gewindekopfes 18 damit eine Kompression aus.

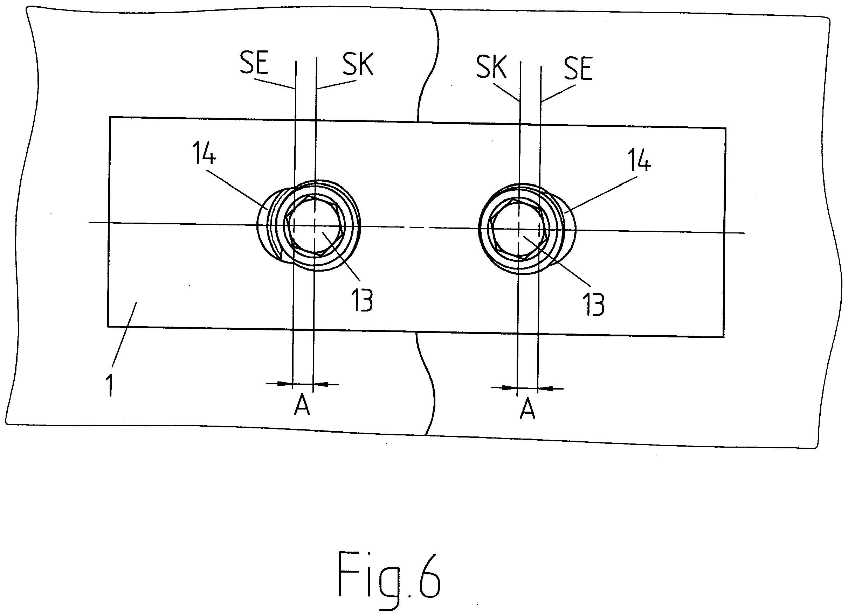

Die Fig. 6 zeigt die vollständig eingeschraubten Knochenschrauben 13.

**Bezugszeichenliste**

| | |
|---|---|
| Knochenplatte | 1 |
| Fixations- und Kompressionslöcher | 2, 3 |
| Kreisförmiges Loch mit D1 | 2.1 |
| Kreisförmiges Loch mit D2 | 2.2 |
| Oberseite von 1 | 4 |

| | |
|---|---|
| Unterseite von 1 | 5 |
| Längsteil von 1 | 6 |
| Endteil von 1 | 7 |
| T-förmige Verbreiterung | 8 |
| Endteil von 1 | 9 |
| L-förmige Verlängerung | 10 |
| Kreisförmige Löcher | 11 |
| Zylindrisches Innengewinde von 11 | 12 |
| Knochenschraube | 13 |
| Sichelförmige Erweiterung | 14 |
| Zylindrisches Innengewinde von 2.1 | 15 |
| Kanten zwischen 2.1 und 14 | 16 |
| Offener Teil von 15 | 17 |
| Gewindekopf von 13 | 18 |
| Vorbohrung | 19 |
| Knochenteile | 20.1, 20.2 |
| Konisches Außengewinde von 18 | 21 |
| Achse von 2.1 | AA |
| Achse von 2.2 | AB |
| Abstand Symmetriezentren | A |
| Bewegungskomponente | K |
| Längsachse von 1 | LA |
| Durchmesser von 2.1 | R1 |
| Durchmesser von 2.2 | R2 |
| Symmetriezentrum von 2.1 | SK |
| Symmetriezentrum von 2.2 | SE |
| Konuswinkel von 18 | α |
| Kreisbogen | BG |

Hierzu 6 Blatt Zeichnungen

## Patentansprüche

1. Vorrichtung zur winkelstabilen Fixation und Kompression einer Bruchstelle bzw. Osteotomie an einem Knochen, mit einer Knochenplatte (1), die aus einer Oberseite (4), einer Unterseite (5) und mehreren die Ober- und Unterseite verbindenden, in Längsachse (LA) der Platte angeordneten Löchern (2) gebildet ist, und Knochenschrauben (13) mit Gewindekopf (18), die in den Löchern (2) aufgenommen und im Knochen verschraubbar sind, wobei mindestens ein Loch (2) eine Kombination aus zwei kreisrunden Löchern (2.1;2.2)) mit unterschiedlich großen Durchmessern (D1,D2) ist und die Symmetriezentren (SK,SE) der beiden Löcher (2.1;2.2) auf der Längsachse (LA) der Knochenplatte (1) angeordnet sind, wobei die kreisrunden Löcher (2.1;2.2) so gegeneinander in Richtung Längsachse (LA) verschoben angeordnet sind, dass ein Kreisabschnitt des kleinen Lochs (2.2) am Umfang des größeren Loches (2.1) eine durch Kanten (16) abgesetzte, einseitige sichelartige Erweiterung (14) bildet, **dadurch gekennzeichnet, dass** die Innenwandung des großen Loches (2.1) mit einem von der Oberseite (4) zur Unterseite (3) verlaufenden Innengewinde (15) versehen ist, das sich bis an die Kanten (16) erstreckt, und dass die sichelartige Erweiterung (14) ohne Gewinde ausgebildet ist und das Symmetriezentrum (SE) des kleinen Loches (2.2) nahe am Symmetriezentrum (SK) des großen Loches (2.1) angeordnet ist, wobei der Abstand (A) der beiden Symmetriezentren (SK; SE) der beiden Löcher (2.1, 2.2) die Bedingung 1/18 D2 < A < 1/3 D2 erfüllt, wobei die Knochenschrauben (13) den Knochen im Symmetriezentrum (SE) des kleinen Loches (2.2) durch Einschrauben fixieren und zugleich der Gewindekopf (18) ein konisches Außengewinde (21) aufweist, das beim Einschrauben das Innengewinde des großen Loches (2.1) erfasst und eine relative Verschiebung der Platte zum Knochen erzeugt.

2. Vorrichtung nach Anspruch 1, **dadurch gekennzeichnet, dass** das größere Loch (2.1) mit Innengewinde (15) zur Bruchstelle bzw. Osteotomie hin und die sichelartige Erweiterung (14) jeweils von der Bruchstelle bzw. Osteotomie weg anordnenbar sind.

3. Vorrichtung nach Anspruch 1, **dadurch gekennzeichnet, dass** die Achsen (AA, AB) des Loches (2.1) und der Erweiterung (14)im Wesentlichen senkrecht zur Längsachse (LA) angeordnet sind und parallel zueinander verlaufen.

4. Vorrichtung nach Anspruch 1, **dadurch gekennzeichnet, dass** das Verhältnis der Durchmesser (D1:D2) der Löcher (2.1;2.2) die Bedingung 0,5 < D2/D1 < 0,8 erfüllt.

5. Vorrichtung nach Anspruch 1 bis 4, **dadurch gekennzeichnet, dass** das Innengewinde (15) der Knochenplatte (1) mit dem Außengewinde (21) des konischen Gewindekopfes (18) abgestimmt ist.

6. Vorrichtung nach Anspruch 1 bis 3, **dadurch gekennzeichnet, dass** das Innengewinde des großen Loches (2.1) ein kreiszylindrisches Feingewinde ist.

7. Vorrichtung nach Anspruch 1 bis 6, **dadurch gekennzeichnet, dass** das konische Außengewinde (21) des Gewindekopfes (18) einen Konuswinkel (α) von 10° bis 16°, vorzugsweise 16° aufweist.

8. Vorrichtung nach Anspruch 1, **dadurch gekennzeichnet, dass** die Vorrichtung eine Bohrlehre zum Setzen einer Bohrung im Symmetriezentrum (SE) der Erweiterung (14) im Knochen umfasst.

9. Vorrichtung nach Anspruch 1, **dadurch gekennzeichnet, dass** Knochenplatte (1) und Knochenschraube (13) aus biokompatiblen Werkstoffen wie Titan, einer Titanlegierung, Kobalt-Chrom-Legierung, Stahl, Kunststoff oder Composite bestehen.

## Claims

1. A device for fixed-angle fixation and compression of a bone fracture or osteotomy, having a bone plate (1) which is formed of a top face (4), a bottom face (5) and a plurality of holes (2) connecting the top face and bottom face and arranged along the longitudinal axis (LA) of the plate, and bone screws (13) with threaded heads (18), which are accommodated in the holes (2) and may be screwed into the bone, at least one hole (2) being a combination of two circular holes (2.1; 2.2) with different-sized diameters (D1, D2) and the centres of symmetry (SK, SE) of the two holes (2.1; 2.2) being arranged on the longitudinal axis (LA) of the bone plate (1), the circular holes (2.1; 2.2) being arranged offset in such a way relative to one another in the direction of the longitudinal axis (LA) that a circular portion of the small hole (2.2) forms a unilateral, crescent-shaped extension (14) demarcated by edges (16) at the circumference of the larger hole (2.1), **characterised in that** the internal wall of the large hole (2.1) is provided with an internal thread (15) running from the top face (4) to the bottom face (3), which internal thread extends up to the edges (16), and **in that** the crescent-shaped extension (14) is unthreaded and the centre of symmetry (SE) of the small hole (2.2) is arranged close to the centre of symmetry (SK) of the large hole (2.1), wherein the distance (A) between the two centres of symmetry (SK; SE) of the two holes (2.1; 2.2) fulfils the condition 1/18 D2 < A < 1/3 D2, wherein the bone screws (13) fix the bone in the centre of symmetry (SE) of the small hole (2.2) by being screwed in and at the same time the threaded head (18) comprises a conical external thread (21) which on screwing in engages the internal thread of the large hole (2.1) and brings about relative displacement of the plate relative to the bone.

2. A device according to claim 1, **characterised in that** respectively the larger hole (2.1) may be arranged with internal thread (15) towards the fracture or osteotomy and the crescent-shaped extension (14) away from the fracture or osteotomy.

3. A device according to claim 1, **characterised in that** the axes (AA, AB) of the hole (2.1) and of the extension (14) are arranged substantially perpendicular to the longitudinal axis (LA) and run parallel to one another.

4. A device according to claim 1, **characterised in that** the ratio of the diameters (D1:D2) of the holes (2.1; 2.2) fulfils the condition 0.5 < D2/D1 < 0.8.

5. A device according to claims 1 to 4, **characterised in that** the internal thread (15) of the bone plate (1) is matched with the external thread (21) of the conical threaded head (18).

6. A device according to claims 1 to 3, **characterised in that** the internal thread of the large hole (2.1) is a circular cylindrical fine-pitch thread.

7. A device according to claims 1 to 6, **characterised in that** the conical external thread (21) of the threaded head (18) has a cone angle (α) of 10° to 16°, preferably 16°.

8. A device according to claim 1, **characterised in that** the device comprises a drill template for placing a bore in the centre of symmetry (SE) of the extension (14) in the bone.

9. A device according to claim 1, **characterised in that** bone plate (1) and bone screw (13) consist of biocompatible materials such as titanium, titanium alloy, cobalt-chromium alloy, steel, plastics or composites.

## Revendications

1. Dispositif de fixation à stabilité angulaire et de compression d'une fracture ou d'une ostéotomie au niveau d'un os, avec une plaque osseuse (1) qui est formée par une face supérieure (4), une face inférieure (5) et plusieurs trous (2) disposés dans l'axe longitudinal (LA) de la plaque et reliant la face supérieure et la face inférieure, et des vis à os (13) avec une tête filetée (18) qui peuvent être logées dans les trous (2) et vissées dans l'os, dans lequel au moins un trou (2) est une combinaison de deux trous circulaires (2.1 ; 2.2) avec des diamètres de tailles différentes (D1, D2) et les centres de symétrie (SK, SE) des deux trous (2.1 ; 2.2) sont disposés sur l'axe longitudinal (LA) de la plaque osseuse (1), dans lequel les trous circulaires (2.1 ; 2.2) sont disposés de manière décalée l'un par rapport à l'autre dans la direction de l'axe longitudinal (LA) de sorte qu'une section circulaire du petit trou (2.2) forme au niveau de la périphérie du trou plus gros (2.1) un élargissement en forme de faucille unilatéral (14) qui se termine par des arêtes (16) **,caractérisé en ce que** la paroi intérieure du gros trou (2.1) est dotée d'un filetage interne (15) allant de la face supérieure (4) à la face inférieure (3), qui se prolonge jusqu'aux arêtes (14), et **en ce que** l'élargissement en forme de faucille (14) est configuré sans filetage et le centre de symétrie (SE) du petit trou (2.2) est disposé à proximité du centre de symétrie (SK) du gros trou (2.1), dans lequel la distance (A) entre les deux centres de symétrie (SK; SE) des deux trous (2.1, 2.2) remplit la condition 1/18 D2 < A < 1/3 D2, dans lequel les vis à os (13) fixent l'os dans le centre de symétrie (SE) du petit trou (2.2) par le biais d'un vissage et en même temps la tête filetée (18) présente un filetage externe conique (21), qui engage par vissage le filetage interne du gros trou (2.1) et génère un décalage relatif de la plaque à l'os.

2. Dispositif selon la revendication 1, **caractérisé en ce que** le trou plus gros (2.1) peut être disposé avec le filetage interne (15) orienté vers la fracture ou l'ostéotomie et l'élargissement en forme de faucille (14) peut être disposé respectivement à l'opposé de la fracture ou ostéotomie.

3. Dispositif selon la revendication 1, **caractérisé en ce que** les axes (AA, AB) du trou (2.1) et de l'élargissement (14) sont disposés pour l'essentiel de manière perpendiculaire à l'axe longitudinal (LA) et s'étendent parallèlement les uns aux autres.

4. Dispositif selon la revendication 1, **caractérisé en ce que** la proportion des diamètres (D1 ; D2) des trous (2.1 ; 2.2) remplit la condition 0,5 < D2/D1 < 0,8.

5. Dispositif selon les revendications 1 à 4, **caractérisé en ce que** le filetage interne (15) de la plaque osseuse (1) concorde avec le filetage externe (21) de la tête filetée conique (18).

6. Dispositif selon les revendications 1 à 3, **caractérisé en ce que** le filetage interne du gros trou (2.1) est un filetage fin cylindrique circulaire.

7. Dispositif selon les revendications 1 à 6, **caractérisé en ce que** le filetage externe conique (21) de la tête filetée (18) présente un angle de cône (α) de 10° à 16°, de préférence de 16°.

8. Dispositif selon la revendication 1, **caractérisé en ce que** le dispositif comprend dans l'os un gabarit de perçage pour l'aménagement d'un alésage dans le centre de symétrie (SE) de l'élargissement (14).

9. Dispositif selon la revendication 1, **caractérisé en ce que** la plaque osseuse (1) et la vis à os (13) se composent de matériaux biocompatibles comme le titane, un alliage de titane, un alliage de cobalt et de chrome, l'acier, une matière plastique ou des composites.
